# EUROPEAN PATENT SPECIFICATION

(11) **EP 2 004 819 B1**
(45) Date of publication and mention of the grant of the patent: **22.07.2015**
(21) Application number: 07754572.1
(22) Date of filing: 03.04.2007
(51) Int. Cl.: C12N 9/90

(54) **FILAMENTOUS FUNGI HAVING REDUCED UDP-GALACTOFURANOSE CONTENT**
FILAMENTÖSE PILZE MIT VERRINGERTEM GEHALT AN UDP-GALAKTOFURANOSE
CHAMPIGNONS FILAMENTEUX À TENEUR EN UDP-GALACTOFURANOSE RÉDUITE

(30) Priority: 05.04.2006 US 789317 P
(43) Date of publication of application: 24.12.2008
(73) Proprietor: Danisco US Inc., Palo Alto, CA 94304 (US); University of Ghent, 9000 Ghent (BE); Flanders Interuniversity Inst. for Biotechnology vzw, 9052 Zwijnaarde, Ghent (BE)
(72) Inventor: WARD, Michael, Palo Alto, California 94304 (US); VERVECKEN, Wouter, 9052 Zwijnaarde (BE); CONTRERAS, Roland, 9052 Zwijnaarde (BE)
(74) Representative: Forrest, Graham Robert
(86) International application number: PCT/US2007/008067
(87) International publication number: WO 2007/123801

(56) References cited:
- WO-A-2007/011221
- SCHMALHORST P ET AL: "UDP-Galactopyranose mutase in Aspergillus fumigatus" ABSTRACTBOOK OF THE FEBS ADVANCED LECTURE COURSE HFP 2005, [Online] May 2005 (2005-05), page 65, P69A, XP002446511 & FEBS Advanced Lecture Course, Human Fungal Pathogens: Molecular Mechanisms of Host-Pathogen Interactions and Virulence, La Colle-sur-Loup, FR; May 21-28, 2005 Retrieved from the Internet: URL:http://www.pasteur.fr/infosci/conf/hfp 2005/Abstract_Book.pdf> [retrieved on 2007-08-09]
- SCHMALHORST P: "Deletion des UDP-Galactopyranose-Mutase-Gens in Aspergillus fumigatus" DIPLOMA THESIS, FACULTY OF CHEMISTRY, UNIVERSITY OF HANNOVER, GERMANY, 2004, XP009088372
- BAKKER H ET AL: "Identification and partial characterization of two eukaryotic UDP-galactopyranose mutases" BIOLOGICAL CHEMISTRY, XX, XX, vol. 386, no. 7, July 2005 (2005-07), pages 657-661, XP009058339 ISSN: 1431-6730

## Description

### BACKGROUND

Galactofuranose (Gal*f*) is a sugar that is absent in mammals but present in a variety of microbes, often within proteins that are glycosylated. The synthesis of UDP-galactofuranose, the source of galactofuranose in glycoproteins, is catalyzed by the enzyme UDP-galactopyranose mutase (UGM) (EC 5.4.99.9), which catalyzes the rearrangement of UDP-galactopyranose (Gal*p*) to UDP-galactofuranose. Using UDP-galactofuranose as a substrate, the enzyme UDP-galactofuranose transferase adds galactofuranose residues to a glycoprotein. Certain proteins secreted by certain filamentous fungi, e.g., *A. niger,* have been shown to have terminal Gal*f* residues (Wallis et al., 2001, Eur J Biochem 268:4134-4143).

This disclosure relates to filamentous fungi having reduced UDP-galactofuranose and filamentous fungi that produce glycoproteins having reduced galactofuranose content.

Literature of interest includes: published U.S. patent applications 20060041113, 20050164351, 20060040353 and 20050208623, and the following references: Beverley et al (Eukaryotic Cell. 2005 June; 4: 1147-1154); Bakker et al (Biol Chem. 2005 386:657-61); Wallis et al (2001, Eur. J. Biochem. 268:4134-4143) and Abstracts 33 and 47 of the 16th Joint Meeting of the Belgian Working Group for Glycosciences, October 27-29,2005.

### SUMMARY OF THE INVENTION

A filamentous fungal cell having reduced UDP-galactofuranose and reduced galactofuranose (Gal*f*) is provided. The fungal cell contains a nuclear genome comprising an inactivated UDP-galactopyranose mutase (UDP-gal*p* mutase) gene and a recombinant nucleic acid for expression of a secreted protein. Also provided are methods of producing a protein using the subject fungal cell, as well as methods of producing the subject fungal cell. In certain embodiments, the UDP-galP mutase gene may contain a deletion, an insertion, or a rearrangement.

The fungal cell may be an *Aspergillus* cell such as an *Aspergillus niger* cell or an *Aspergillus oryzae* cell.

In certain embodiments, the protein may be a glycosylated protein. The glycosylated protein may be heterologous to the cell, native to the cell. In certain embodiments, the protein may be a therapeutic protein, an antibody protein or an enzyme such as an amylase, protease, cellulase, xylanase or phytase, for example.

The nuclear genome may comprise at least two inactivated UDP-galactopyranose mutase genes.

The protein may have reduced galactofuranose content, as compared to the protein produced in an equivalent cell comprising a nuclear genome comprising an intact UDP-galactopyranose mutase gene.

In certain embodiments, the cell may further comprise a recombinant nucleic acid for expression of a mannosidase.

A method of producing a protein, comprising: culturing the above-recited cell in culture medium under conditions suitable to produce the protein is also provided. The protein may, in certain embodiments, be a glycosylated protein, a therapeutic protein, or an enzyme, for example.

The method may further comprise: recovering the protein from the culture medium.

A method of making a subject cell, comprising: a) introducing a recombinant nucleic acid into a filamentous fungal cell so that the recombinant nucleic acid recombines with the UDP-galactopyranose mutase gene of the genome of the cell; and b) introducing a second recombinant nucleic acid into the cell that provides for expression of the secreted protein, is also provided.

In certain embodiments, the nucleic acid inserts into the UDP-galactopyranose mutase gene.

The nucleic acid may delete at least a portion of the UDP-galactopyranose mutase gene.

### BRIEF DESCRIPTION OF THE DRAWINGS

Certain aspects of the following detailed description are best understood when read in conjunction with the accompanying drawings. It is emphasized that, according to common practice, the various features of the drawings are not to-scale. On the contrary, the dimensions of the various features are arbitrarily expanded or reduced for clarity. Included in the drawings are the following figures:
**Fig. 1** shows data from DNA-sequencer-aided fluorophore-assisted electrophoresis (DSA-FACE) analysis of glycans of secreted proteins from parental strain.
**Fig. 2** schematically illustrates vector construction of mannosidase constructs.
**Fig. 3** shows data from DSA-FACE analysis of glycans of different ManHDEL transformants.
**Fig. 4** shows data from DSA-FACE analysis of glycans of transformant 2.
**Fig. 5** schematically shows the strategy used for inactivating a UDP-Gal*p* mutase gene.
**Figs. 6a** **and** **6b** schematically show the vector construction scheme for inactivating a UDP-Gal*p* mutase gene.
**Fig. 7** shows the N-glycan profiles of 6 of the 140 mutase knockout candidates that were analyzed.
**Fig. 8****:** shows data from DSA-FACE analysis of glycans of secreted proteins of putative glf mutants.
**Fig. 9** schematic illustrates the genome at the UDP-Gal*p* mutase locus before and after the integration of the deletion fragments.
**Fig. 10** shows results of Southern analysis of UDP-Gal*p* mutase deleted *A. niger* strains.
**Fig. 11** shows data from glycan analysis of clone 2F65.
**Fig. 12** *A*. *nidulans* UDP-Gal*p* mutase gene sequence (top; SEQ ID NO:1) and *A. nidulans* UDP-Galp mutase predicted amino acid sequence (bottom; SEQ ID NO:2)
**Fig. 13** shows the unconfirmed DNA sequence (SEQ ID NO:3) and predicted amino acid sequence (SEQ ID NO:4) of one *A. niger* UDP- Gal*p* mutase homolog.
**Fig. 14** shows the sequence of an *A. niger* UDP- Gal*p* mutase ORF (SEQ ID NOS:5 and 6).
**Fig. 15** shows the sequence of an *A. niger* UDP-Gal*p* mutase gene with introns (SEQ ID NO:7).
**Fig. 16** shows corrected *A. niger* UDP-Gal*p* mutase sequences (SEQ ID NO:8-10).
**Fig. 17** shows the sequence of second *A. niger* UDP-Gal*p* mutase gene (SEQ ID NO:11).

### DEFINITIONS

Unless defined otherwise, all technical and scientific terms used herein have the same meaning as commonly understood by one of ordinary skill in the art to which this invention belongs. Singleton, et al., DICTIONARY OF MICROBIOLOGY AND MOLECULAR BIOLOGY, 2D ED., John Wiley and Sons, New York (1994), and Hale & Markham, THE HARPER COLLINS DICTIONARY OF BIOLOGY, Harper Perennial, N.Y. (1991) provide one of skill with the general meaning of many of the terms used herein. Still, certain terms are defined below for the sake of clarity and ease of reference.

The term "recombinant" when used in reference to a cell, nucleic acid, protein or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express nucleic acids or polypeptides that are not found within the native (non-recombinant) form of the cell or express native genes that are otherwise abnormally expressed, under expressed, over expressed or not expressed at all.

The terms "protein" and "polypeptide" are used interchangeably herein. The conventional one-letter or three-letter code for amino acid residues is used herein.

The term "antibody protein" refers to a protein containing one or more polypeptides that specifically binds an antigen. Included by this term are antibodies of any isotype, fragments of antibodies which retain specific binding to antigen, including, but not limited to, Fab, Fv, scFv, Fd, Fab', Fv, F(ab')₂ antibodies, antibody fragments that retain specific binding to antigen, monoclonal antibodies, chimeric antibodies, humanized antibodies, single-chain antibodies, bi-functional (i.e. bi-specific) hybrid antibodies and fusion proteins comprising an antigen-binding portion of an antibody and a non-antibody protein.

A "signal sequence" means a sequence of amino acids present at the N-terminal portion of a protein which facilitates the secretion of the mature form of the protein outside the cell. The definition of a signal sequence is a functional one. The mature form of the extracellular protein lacks the signal sequence which is cleaved off during the secretion process.

A "gene" refers to a DNA segment that is involved in producing a polypeptide and includes regions preceding and following the coding regions, e.g., the promoter and terminator, as well as intervening sequences (introns) between individual coding segments (exons).

An "inactivated gene" is a locus of a genome that, prior to its inactivation, was capable of producing a protein, i.e., capable of being transcribed into an RNA that can be translated to produce a full length polypeptide. The gene is inactivated in that it does not produce a full length protein. A gene may be inactivated by altering a sequence required for its transcription, by altering a sequence required for RNA processing, e.g., splicing, by altering a sequence required for translation, for example, a deleted gene, a gene containing a deleted region, a gene containing a rearranged region, and a gene containing an insertion are types of inactivated gene. A gene may also be inactivated using RNAi, antisense, or any other method that abolishes gene expression.

The term "nucleic acid" encompasses DNA, RNA, single stranded or double stranded and chemical modifications thereof. The terms "nucleic acid" and "polynucleotide" may be used interchangeably herein. Because the genetic code is degenerate, more than one codon may be used to encode a particular amino acid, and the present invention encompasses polynucleotides which encode a particular amino acid sequence.

A "vector" refers to a polynucleotide sequence designed to introduce nucleic acids into one or more cell types. Vectors include cloning vectors, expression vectors, shuttle vectors, plasmids, phage particles, cassettes and the like.

An "expression vector" as used herein means a DNA construct comprising a DNA sequence which is operably linked to a suitable control sequence capable of effecting expression of a protein in a suitable host. Such control sequences may include a promoter to effect transcription, an optional operator sequence to control transcription, a sequence encoding suitable ribosome binding sites on the mRNA, enhancers and sequences which control termination of transcription and translation.

A "promoter" is a regulatory sequence that is involved in binding RNA polymerase to initiate transcription of a gene.

"Under transcriptional control" is a term well understood in the art that indicates that transcription of a polynucleotide sequence, usually a DNA sequence, depends on its being operably linked to an element which contributes to the initiation of, or promotes transcription.

"Under translational control" is a term well understood in the art that indicates a regulatory process which occurs after mRNA has been formed.

As used herein when describing proteins and genes that encode them, the term for the gene is capitalized and is italicized, (e.g., the gene that encodes a UDP-galactopyranose mutase may be denoted as *UGM*)*.* The term for the protein is generally not italicized and capitalized, (e.g., the protein encoded by the *UGM* gene may be denoted as UGM).

The term "operably linked" refers to juxtaposition wherein the elements are in an arrangement allowing them to be functionally related. For example, a promoter is operably linked to a coding sequence if it controls the transcription of the sequence.

The term "selective marker" refers to a protein capable of expression in a host that allows for ease of selection of those hosts containing an introduced nucleic acid or vector. Examples of selectable markers include but are not limited to antimicrobials (e.g., hygromycin, bleomycin, or chloramphenicol) and/or genes that confer a metabolic advantage, such as a nutritional advantage on the host cell.
The term "derived" encompasses the terms "originated from", "obtained" or "obtainable from", and "isolated from".

"Host strain" or "host cell" means a suitable host for an expression vector or DNA construct comprising a polynucleotide encoding a polypeptide. In specific embodiments, the host strains may be a filamentous fungal cell. The term "host cell" includes both cells and protoplasts.

The term "filamentous fungi" refers to all filamentous forms of the subdivision Eumycotina (See, Alexopoulos, C. J. (1962), INTRODUCTORY MYCOLOGY, Wiley, New York). These fungi are characterized by a vegetative mycelium with a cell wall composed of chitin, glucans, and other complex polysaccharides. The filamentous fungi of the present invention are morphologically, physiologically, and genetically distinct from yeasts. Vegetative growth by filamentous fungi is by hyphal elongation and carbon catabolism is obligatory aerobic.

A "non-pathogenic" filamentous fungi is a strain that is not pathogenic to humans.

The term "culturing" refers to growing a population of microbial cells under suitable conditions in a liquid or solid medium.

The term "heterologous" with reference to a polynucleotide or protein refers to a polynucleotide or protein that does not naturally occur in a host cell. In some embodiments, the protein is a commercially important industrial protein. It is intended that the term encompass proteins that are encoded by naturally occurring genes, mutated genes, and/or synthetic genes. The term "homologous" with reference to a polynucleotide or protein refers to a polynucleotide or protein that occurs naturally in the host cell.

The terms "recovered", "isolated", and "separated" as used herein refer to a protein, cell, nucleic acid or amino acid that is removed from at least one component with which it is naturally associated.

As used herein, the terms "transformed", "stably transformed" and "transgenic" used in reference to a cell means the cell has a non-native (e.g., heterologous) nucleic acid sequence integrated into its genome or as an episomal plasmid that is maintained through multiple generations.

As used herein, the term "expression" refers to the process by which a polypeptide is produced based on the nucleic acid sequence of a gene. The process includes both transcription and translation.

The term "introduced" in the context of inserting a nucleic acid sequence into a cell, means "transfection", or "transformation" or "transduction" and includes reference to the incorporation of a nucleic acid sequence into a eukaryotic or prokaryotic cell wherein the nucleic acid sequence may be incorporated into the genome of the cell (e.g., chromosome, plasmid, plastid, or mitochondrial DNA), converted into an autonomous replicon, or transiently expressed (e.g., transfected mRNA).

As used herein the term "specific activity" means an enzyme unit defined as the number of moles of substrate converted to product by an enzyme preparation per unit time under specific conditions. Specific activity is expressed as units (U)/mg of protein.

The term "glycosylation" refers to the post-transcriptional modification of a protein by the addition of carbohydrate moieties, wherein the carbohydrate is either N-linked or O-linked resulting in a glycoprotein. An N-linked carbohydrate moiety of a glycoprotein is attached by a glycosidic bond to the β-amide nitrogen of an asparagine residue. An O-linked carbohydrate is attached by a glycosidic bond to a protein through the hydroxy group of a serine or a threonine residue.

The term "equivalent fungal cell" refers to a fungal cell grown under essentially the same conditions as a subject fungal cell and which does not include an inactivated UDP-galactofuranose mutase gene. In some examples, the equivalent fungal cell has not been altered using the methods described below.

The term "hybridization" refers to the process by which a strand of nucleic acid joins with a complementary strand through base pairing as known in the art. A nucleic acid is considered to be "Selectively hybridizable" to a reference nucleic acid sequence if the two sequences specifically hybridize to one another under moderate to high stringency hybridization and wash conditions. Moderate and high stringency hybridization conditions are known (see, e.g., Ausubel, et al., Short Protocols in Molecular Biology, 3rd ed., Wiley & Sons 1995 and Sambrook et al., Molecular Cloning: A Laboratory Manual, Third Edition, 2001 Cold Spring Harbor, N.Y.).. One example of high stringency conditions include hybridization at about 42C in 50% formamide, 5X SSC, 5X Denhardt's solution, 0.5% SDS and 100 ug/ml denatured carrier DNA followed by washing two times in 2X SSC and 0.5% SDS at room temperature and two additional times in 0.1 X SSC and 0.5% SDS at 42C.
Other definitions of terms may appear throughout the specification.

### DETAILED DESCRIPTION

A filamentous fungal cell having reduced UDP-galactofuranose is provided. The fungal cell contains a nuclear genome comprising an inactivated UDP-galactopyranose mutase (UDP-gal*p* mutase) gene and a recombinant nucleic acid for expression of a secreted protein. Also provided are methods of producing a protein using the subject fungal cell, as well as methods of producing the subject fungal cell.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Although any methods and materials similar or equivalent to those described herein can be used in the practice or testing of the present invention, exemplary and preferred methods and materials are now described.

It must be noted that as used herein and in the appended claims, the singular forms "a", "an", and "the" include plural referents unless the context clearly dictates otherwise. Thus, for example, reference to "a gene" includes a plurality of such candidate agents and reference to "the cell" includes reference to one or more cells and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### HOST CELLS

As noted above, a recombinant filamentous fungal cell having reduced UDP-galactofuranose is provided. In general terms, the subject fungal cell may have less than about 50%, e.g., less than about 40%, less than about 30%, less than about 20%, or less than about 10% of the UDP-galactofuranose of an equivalent fungal cell. In certain embodiments, the subject cell may have less than about 5% of the UDP-galactofuranose of an equivalent fungal cell. In other embodiments, UDP-galactofuranose may be undetectable in the subject fungal cell. The presence of UDP galactofuranose may be assessed by known methods, e.g., the method of Lee et al, Anal Biochem. 1996 242:1-7.

In particular embodiments, the subject cell may secrete a glycosylated protein having a reduced galactofuranose content (e.g., less than about 50%, e.g., less than about 40%, less than about 30%, less than about 20%, less than about 10%, or less than about 5%), as compared to the same protein secreted by an equivalent fungal cell. In certain embodiments, galactofuranose may be undetectable in the secreted protein. In other words, in certain embodiments a glycosylated protein secreted by the subject fungal cell may contain reduced or no galactofuranose, as compared the same protein secreted by an equivalent fungal cell that has not been altered using the instant methods. The galactofuranose content of a secreted glycoprotein may be assessed by established methods, e.g., using the DSA-FACE method of Callewaert et al. (Glycobiology 2001 11:275-281), or by the methods of Hemming et al (Anal Biochem. 2000 279:136-41) or Groisman et al (Eur. J. Biochem. 1987 65:327-32). The protein secreted by the cell may be a protein that is endogenous to the cell (i.e., a native protein), or a protein that is not native to the cell (i.e., a recombinant protein). In certain embodiments, the protein may be an antibody protein such as a monoclonal antibody, or another protein that is glycosylated by the fungal cell prior to secretion. Examples of such proteins are set forth below.

The subject fungal cell is made by inactivating a UDP-galactopyranose mutase (UGM) gene in the cell, where, as noted above, UDP-galactopyranose mutase is the enzyme that catalyzes the rearrangement of UDP-galactopyranose (Galp) to UDP-galactofuranose (Gal*f*) prior to transfer of the galactofuranose residue onto a protein. The UGM protein may be referred to as UDP-galactopyranose:UDP-galactofuranose mutase or UDP-galactofuranose mutase in other literature. The UGM protein described herein has an activity described as EC 5.4.99.9, according to IUMBM enzyme nomenclature.

In certain embodiments therefore, a subject cell may have reduced (e.g., undetectable) UDP-galactopyranose mutase activity (e.g., less than about 50%, e.g., less than about 40%, less than about 30%, less than about 20%, less than about 10%, or less than about 5% of the UDP-galactopyranose mutase activity) as compared to an equivalent fungal cell that has not been altered using the method described below. UDP-galactopyranose mutase activity assays may be determined using the methods of Beverley et al (Eukaryotic Cell. 2005 June; 4: 1147-1154).

Genes encoding UDP-galactopyranose mutase are termed *UGM* genes herein. Such genes may be referred to as *GLF* genes in some disclosures. A fungal cell that has not been altered by the methods described herein may contain one, two or three or more *UGM* genes. In certain embodiments, therefore, a fungal cell containing one or more (e.g., one, two or three or more) inactivated *UGM* genes is provided, depending on the number of *UGM* genes present in the genome of the cell.

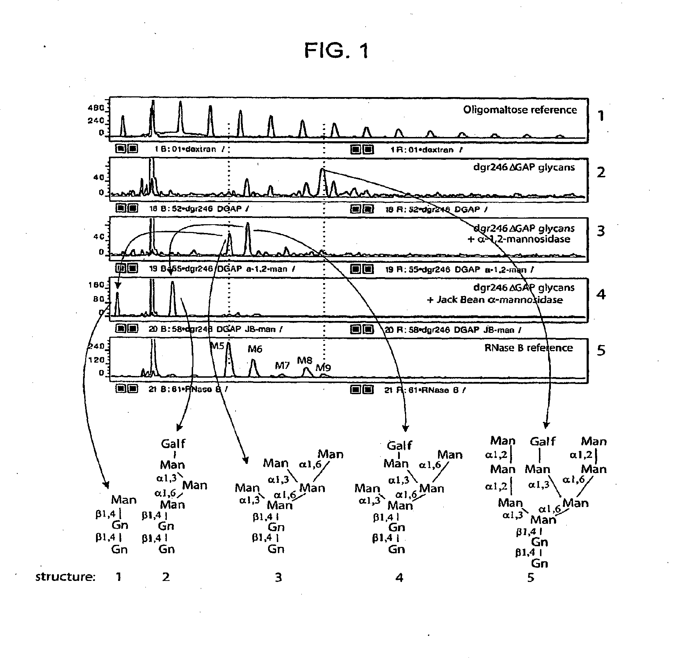

The DNA sequences of several fungal *UGM* genes and the proteins encoded by those genes have been determined and deposited into NCBI's Genbank database. Further, several conserved domains of UDP-galactopyranose mutase have been identified, as well as a number of conserved amino acids, allowing the identification of further fungal *UGM* genes by bioinformatic methods (see, e.g., Fig. 1 of Bakker, *supra* and Fig. 1 of Beverley, *supra*). Methods for identifying *UGM* genes in filamentous fungi are set forth in Beverley et al *supra* and Bakker et al, *supra.*

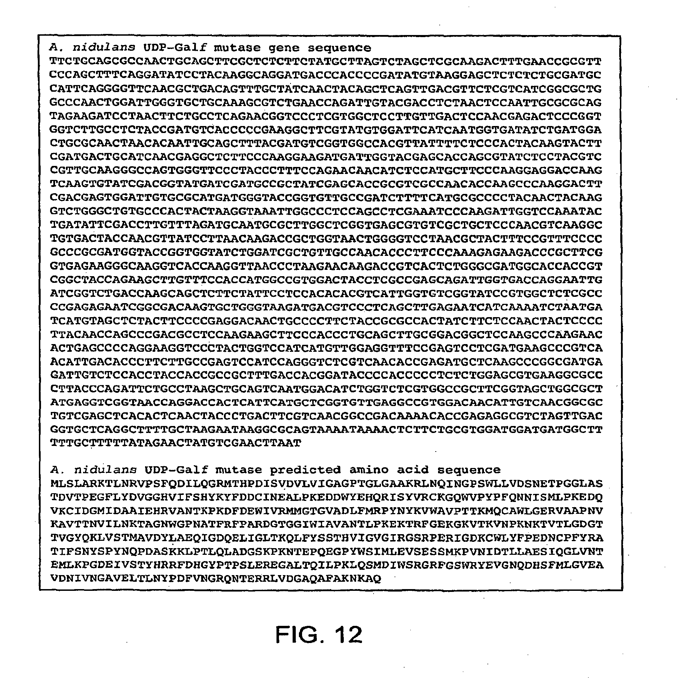

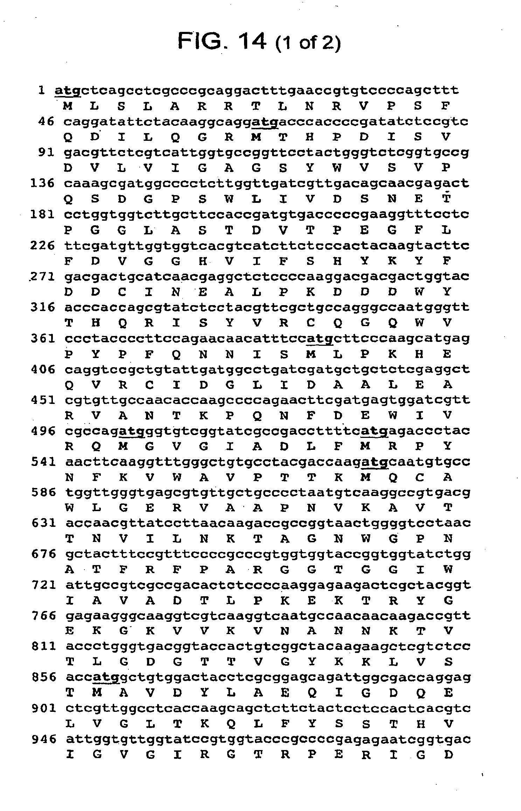

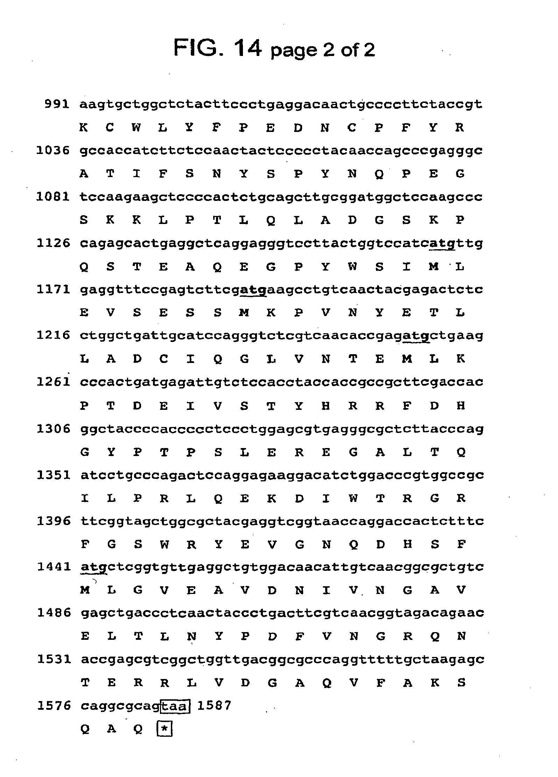

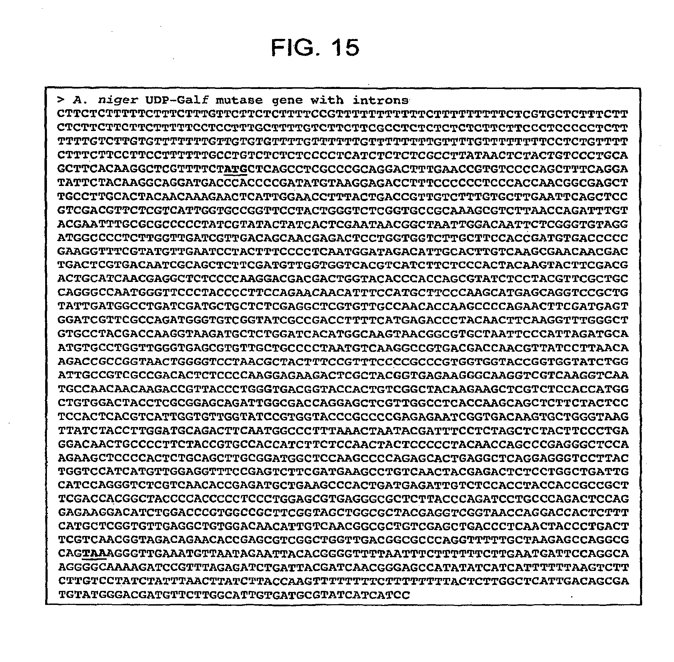

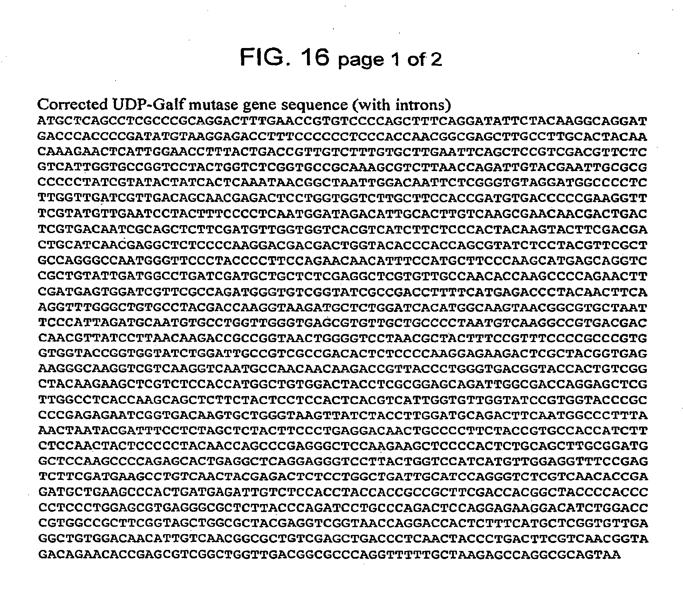

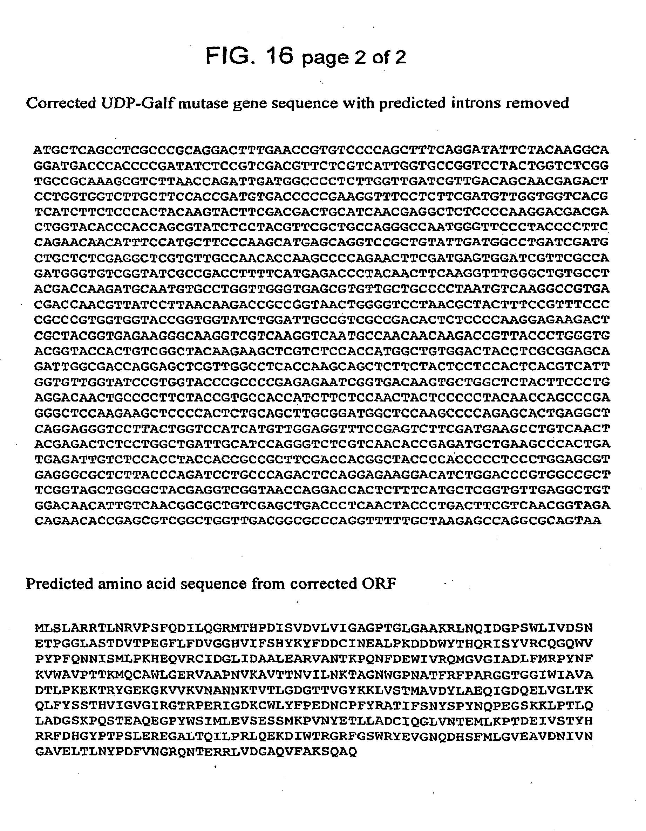

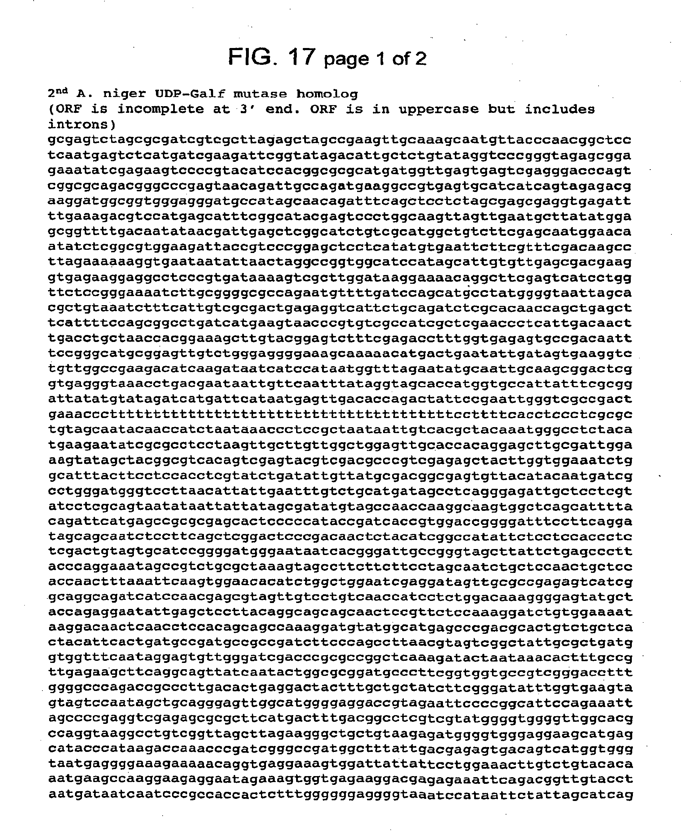

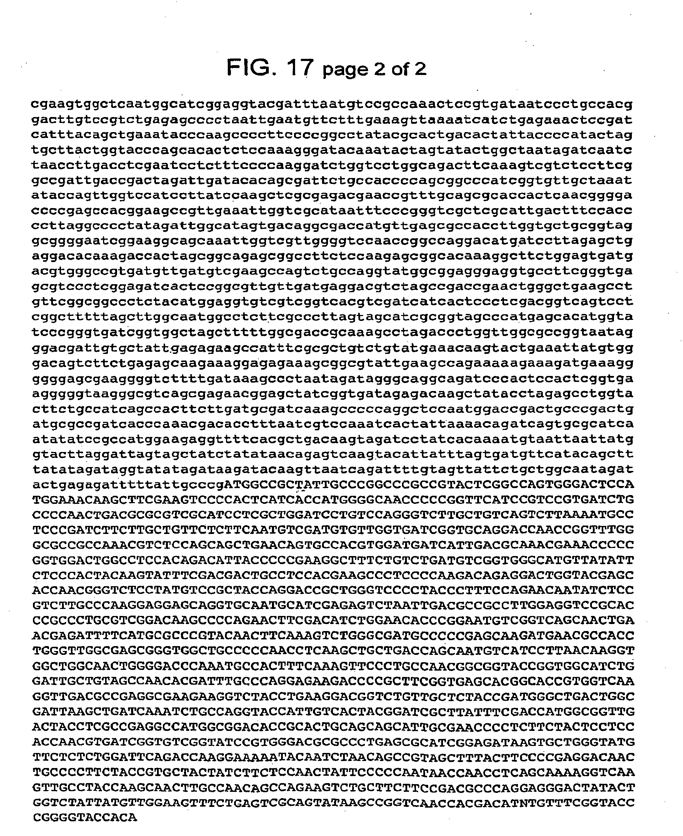

An *UGM* gene may have: a) at least 70% (e.g., at least 80%, at least 90%, at least 95%, at least 97% or at least 98% sequence identity) to a *UGM* sequence deposited in NCBI's Genbank database or depicted in Figs. 12-17; b) may hybridize under stringent conditions to a *UGM* sequence deposited in NCBI's Genbank database or depicted in Figs 12-17; or c) may encode a polypeptide that has at least 70% (e.g., at least 80%, at least 90%, at least 93%, at least 95%, at least 97% or at least 98% sequence identity) to a UGM sequence deposited in NCBI's Genbank database or depicted in Figs 12-17. Exemplary UGM and UGM sequence deposited in NCBI's Genbank database include: GID:67008315 (accession number AJ871145.2; *Aspergillus fumigatus* mRNA for UDP-galactopyranose mutase); GID:70999265 (Accession no.: XM_749259.1; *Aspergillus fumigatus* Af293 hypothetical protein Afu3g12690); GID:67525308 (accession no.: XM_655624.1; *Aspergillus nidulans* FGSC A4 hypothetical protein AN3112.2), GID:85080821 (accession no.: XM_951515.1; *Neurospora crassa* N150 hypothetical protein NCU01824.1 mRNA); GID:32415568 (accession no. XM_328262.1; *Neurospora crassa* strain OR74A); GID:84573902 (accession no.: *AB226201.1; Aspergillus oryzae* cDNA, contig sequence: AoEST3060); GID:71018020 (accession no.: XM_754148.1; *Ustilago maydis* 521 hypothetical protein UM03094.1 mRNA); GID:59859950 (accession no.: AY900624.1 *Filobasidiella neoformans* UDP-galactopyranose mutase (GLF) mRNA); *Aspergillus nidulans* (accession no.: EAA63683); *Neurospora crassa* (accession no.: EAA27372); *Magneporthe grisea* (accession no.: EAA55038); *Gibberella zeae* (accession no.: EAA75642); *Cryptococcus neoformans* (accession no.:EAL19520); *Ustilago maydis* (accession no.: UM03094) and GID:5826675 (accession no.: AL112056.1; CNS019SW *Botrytis cinerea* strain T4 cDNA).

A subject fungal cell may be constructed using any convenient method, for example, by altering the sequence of the *UGM* gene of the cell by making an insertion, deletion, replacement, or rearrangement in the gene for example. The portion of the gene to be altered may be, for example, the coding region or a regulatory element required for expression of the coding region. An example of such a regulatory or control sequence of a gene may be a promoter sequence or a functional part thereof, i.e., a part which is necessary for expression of the gene.

In one embodiment, the subject fungal cell may be constructed by gene deletion methods. Gene deletion techniques enable the partial or complete removal of the gene thereby eliminating their expression. In such methods, the deletion of the gene may be accomplished by homologous recombination using a plasmid that has been constructed to contiguously contain the 5' and 3' regions flanking the gene.

The subject fungal cell may be constructed by introducing, substituting, and/or removing one or more nucleotides in the gene or a regulatory element thereof required for the transcription or translation thereof. For example, nucleotides may be inserted or removed so as to result in the introduction of a stop codon, the removal of the start codon, removal of a splice cite, or a frame-shift of the open reading frame. Such a modification may be accomplished by site-directed mutagenesis or PCR generated mutagenesis in accordance with methods known in the art. See, for example, Botstein and Shortle, 1985, Science 229: 4719; Lo et al., 1985, Proceedings of the National Academy of Sciences USA 81: 2285; Higuchi et al., 1988, Nucleic Acids Research 16: 7351; Shimada, 1996, Meth. Mol. Biol. 57: 157; Ho et al., 1989, Gene 77: 61; Horton et al., 1989, Gene 77: 61; and Sarkar and Sommer, 1990, BioTechniques 8: 404.

The subject fungal cell may be constructed by gene disruption techniques by inserting into the gene of interest an integrative plasmid containing a nucleic acid fragment homologous to the gene which will create a duplication of the region of homology and incorporate vector DNA between the duplicated regions. Such gene disruption can eliminate gene expression if the inserted vector separates the promoter of the gene from the coding region or interrupts the coding sequence such that a non-functional gene product results. A disrupting construct may be simply a selectable marker gene accompanied by 5' and 3' regions homologous to the gene. The selectable marker enables identification of transformants containing the disrupted gene.

The subject fungal cell may be constructed by the process of gene conversion (see, for example, Iglesias and Trautner, 1983, Molecular General Genetics 189: 73-76). For example, in the gene conversion method, a nucleotide sequence corresponding to the gene(s) is mutagenized in vitro to produce a defective nucleotide sequence which is then transformed into the parent strain to produce a defective gene. By homologous recombination, the defective nucleotide sequence replaces the endogenous gene.

The subject fungal cell may be constructed using random or specific mutagenesis using methods that include, but are not limited to, chemical mutagenesis (see, for example, Hopwood, The Isolation of Mutants in Methods in Microbiology (J. R. Norris and D. W. Ribbons, eds.) pp 363-433, Academic Press, New York, 1970) and insertional mutagenesis, such as transposition (see, for example, Youngman et al., 1983, Proc. Natl. Acad. Sci. USA 80: 2305-2309). Modification of the gene may be performed by subjecting the parent strain to mutagenesis and screening for mutant strains in which expression of the gene has been reduced or eliminated. The mutagenesis, which may be specific or random, may be performed, for example, by use of a suitable physical or chemical mutagenizing agent, for example.

Examples of a physical or chemical mutagenizing agent suitable for the present purpose include ultraviolet (UV) irradiation, hydroxylamine, N-methyl-N'-nitro-N-nitrosoguanidine (MNNG), N-methyl-N'-nitrosogaunidine (NTG) O-methyl hydroxylamine, nitrous acid, ethyl methane sulphonate (EMS), sodium bisulphite, formic acid, and nucleotide analogues. When such agents are used, the mutagenesis is typically performed by incubating the parent strain to be mutagenized in the presence of the mutagenizing agent of choice under suitable conditions, and selecting for mutants exhibiting reduced or no expression of a gene.

As noted above, the subject fungal cell is a filamentous fungal cell. The cell may be non-pathogenic, i.e., non-pathogenic to humans. The cells may be filamentous fungal cells of a strain that has a history of use for production of proteins that has GRAS status, i.e., a Generally Recognized as Safe, by the FDA.

The subject fungal cell may be a cell of the following species: Trichoderma, (e.g., Trichoderma reesei (previously classified as T. longibrachiatum and currently also known as *Hypocrea jecorina*), *Trichoderma viride, Trichoderma koningii,* and *Trichoderma harzianum)*); *Penicillium* sp., *Humicola* sp. (e.g., *Humicola insolens* and *Humicola grisea*); *Chrysosporium* sp. (e.g., C. *lucknowense*), *Gliocladium* sp., *Aspergillus* sp. (e.g., *Aspergillus oryzae, Aspergillus niger, Aspergillus nidulans, Aspergillus kawachi, Aspergillus aculeatus, Aspergillus japonicus, Aspergillus sojae, and Aspergillus awamori*), *Fusarium* sp., *Neurospora* sp., *Hypocrea* sp., or *Emericella* sp. (See also, Innis et al., (1985) Sci. 228:21-26), among others. Subject fungal cells may be strains of *Aspergillus niger* which include ATCC 22342, ATCC 44733, ATCC 14331 and strains derived therefrom. A host cell may be one wherein native genes have been deleted or inactivated. For example genes corresponding to protease genes or genes corresponding to cellulase genes.

The subject fungal cell contains a recombinant nucleic acid for expression of a secreted protein in the cell. The protein may be not native to the cell (i.e., heterologous) or native to the cell (i.e., endogenous to the cell). The protein may be expressed using a number of different protocols, e.g., by use of an expression cassette for production of the protein, by operably linking a nucleic acid encoding the protein to a promoter that is part of the genome of the cell with another promoter, or by replacing the promoter that is part of the genome of the cell, for example.

In one embodiment, the protein may be, for example, an enzyme, e.g., a so-called "industrial enzyme", or a protein having therapeutic activity such an antibody.

The nucleic acid encoding a protein may be operably linked to a suitable promoter. The promoter may be derived from genes encoding proteins either homologous or heterologous to the host cell. The promoter may be a truncated or hybrid promoter. Further the promoter may be an inducible or constitutive promoter. Preferably the promoter is useful in an Aspergillus or Trichoderma host. Suitable nonlimiting examples of promoters include *cbh*1, *cbh*2, *egl*1, *pep*A*, hfb*1, *hfb*2, *xyn*1 and *amy.* The promoter may be a *Trichoderma reesei* cbh1 promoter. The promoter may be derived from the genes encoding an *Aspergillus awamori* or *Aspergillus niger* glucoamylase *(glaA)* (Nunberg et al., (1984) Mol.Cell Biol. 4:2306 - 2315 and Boel et al., (1984) EMBO J. 3:1581 - 1585), an *Aspergillus niger* alpha amylase, an *Aspergillus oryzae* TAKA amylase or a *Rhizomucor miehei* aspartic proteinase.

A vector comprising a nucleic acid encoding encoding a protein of interest may include a selectable marker. Examples of selectable markers include but are not limited to ones that confer antimicrobial resistance (e.g. hygromycin, bleomycin, chloroamphenicol and phleomycin). Genes that confer metabolic advantage, such as nutritional selective markers also find use. Some of these markers include amdS, argB and pyr4. Reference is made to Kelley et al., (1985) EMBO J. 4: 475 - 479; Penttila et al., (1987) Gene 61:155 -164 and Kinghorn et al (1992) Applied Molecular Genetics of Filamentous Fungi, Blackie Academic and Professional, Chapman and Hall, London.

The subject fungal cell may be engineered to produce a carbohydrase, such as a liquefying and saccharifying α-amylase, an alkaline α-amylase, a β-amylase, a cellulase; a dextranase, an α-glucosidase, an α-galactosidase, a glucoamylase, a hemicellulase, a pentosanase, a xylanase, an invertase, a lactase, a naringanase, a pectinase or a pullulanase; a protease such as an acid protease, an alkali protease, bromelain, ficin, a neutral protease, papain, pepsin, a peptidase, rennet, rennin, chymosin, subtilisin, thermolysin, an aspartic proteinase, or trypsin; a lipase or esterase, such as a triglyceridase, a phospholipase, a pregastric esterase, a phosphatase, a phytase, an amidase, an iminoacylase, a glutaminase, a lysozyme, or a penicillin acylase; an isomerase such as glucose isomerase; an oxidoreductases, e.g., an amino acid oxidase, a catalase, a chloroperoxidase, a glucose oxidase, a hydroxysteroid dehydrogenase or a peroxidase; a lyase such as a acetolactate decarboxylase, a aspartic β-decarboxylase, a fumarese or a histadase; a transferase such as cyclodextrin glycosyltranferase; or a ligase, for example. The protein may be an aminopeptidase, a carboxypeptidase, a chitinase, a cutinase, a deoxyribonuclease, an α-galactosidase, a β-galactosidase, a β-glucosidase, a laccase, a mannosidase, a mutanase, a pectinolytic enzyme, a polyphenoloxidase, ribonuclease or transglutaminase.

In other embodiments, the protein may be a therapeutic protein such as a glycosylated therapeutic protein (i.e., a protein having a therapeutic biological activity that would be glycosylated in an equivalent cell, e.g., an equivalent cell not altered by the methods described above). Examples of suitable target glycoproteins which may be produced using a subject cell include: erythropoietin, cytokines such as interferon-α, interferon-β, interferon-y, interferon-o, and granulocyte-CSF, GM-CSF, coagulation factors such as factor VIII, factor IX, and human protein C, antithrombin III, thrombin, soluble IgE receptor α-chain, IgG, IgG fragments, IgG fusions, IgM, IgA, interleukins, urokinase, chymase, and urea trypsin inhibitor, IGF-binding protein, epidermal growth factor, growth hormone-releasing factor, annexin V fusion protein, angiostatin, vascular endothelial growth factor-2, myeloid progenitor inhibitory factor-1, osteoprotegerin, .alpha.-1-antitrypsin, α-feto proteins, DNase II, kringle 3 of human plasminogen, glucocerebrosidase, TNF binding protein 1, follicle stimulating hormone, cytotoxic T lymphocyte associated antigen 4-Ig, transmembrane activator and calcium modulator and cyclophilin ligand, soluble TNF receptor Fc fusion, glucagon like protein 1 and IL-2 receptor agonist. Monoclonal antibodies are of particular interest.

The recombinant protein (i.e., non-native) produced by the cell is secreted from the cell into culture media. As such, the cell may further contain a recombinant nucleic acid encoding a fusion polypeptide containing a signal sequence, a protease cleavage site and the protein. The signal sequence may be one is naturally associated with the polypeptide to be expressed.

In addition to the above-described non-native protein, a subject cell may be further engineered to contain a recombinant nucleic acid for expression of non-native enzyme involved in glycan metabolism. For example, the cell may further express an enzyme for adding monosaccharide residues to a glycosylated protein, removing monosaccharide residues from a glycosylated protein, altering monosaccharide residues on a glycosylated protein, or biosynthesis of a particular monosaccharide. In order to "trim back" mannose resides, the cell may be engineered to produce a mannosidase (e.g., α-1,2-mannosidase I, mannosidase II, mannosidase IIx, class III mannosidase or an α-mannosidase from *T. reesei*) to release terminal mannose residues from a protein. In order to transfer other monosaccharides onto the protein, a glycan transferase may be employed. As such, a) a fucosyltransferase, e.g., an α1,2 , α 1,3/4 or α 1,3 fucosyltransferase; b) a galactosyltransferase, e.g., an α1,3 β1,4 galactosyltransferase; c) an N-acetylgalactosaminyltransferase, e.g., an α1,3, β1,4 α1 or Thr polypeptide acetylgalactosaminyltransferase; d) an N-acetylglucosaminyltransferase, e.g., a β1,2, β1,4, β1,6, GPT or hyaluronan synthetase N-acetylglucosaminyltransferase; e) a sialyltransferase, e.g., a α2,6, α2,3, α2,8 or STX sialyltransferase; f) a glucosyltransferase, e.g., a AIg8 or AIg5 glucosyltransferase; or g) a mannosyltransferase such as an α1,2, α1,3, β1,4, Dpm1, Och1 or Pmt mannosyltransferase may be expressed in the cell. Further description of exemplary transferases is found in published U.S. patent application 20050164351.

If such a glycosylation-related enzyme is expressed in a subject cell, it may be targeted to the endoplasmic reticulum (ER), to the golgi apparatus or to other membrane bound components of the secretory apparatus.

Methods of expressing proteins in filamentous fungi, including methods in which cells are engineered to produce secreted protein include those described in U.S. Pats. 6,022,725; 6,268,328; and published U.S. patent applications 20060041113, 20060040353, 20060040353 and 20050208623.

In addition, general methods for the transformation of *Aspergillus* strains are disclosed in Cao et al., (Protein Sci. 2000 9:991 - 1001) and Yelton et al., (Proc. Natl. Acad. Sci. 1984 USA 81: 1470 - 1474) and general methods for the transformation of *Trichoderma* strains are disclosed in Nevalainen et al., (The Molecular Biology of Trichoderma and its Application to the Expression of Both Homologous and Heterologous Genes" in Molecular Industrial Mycology, Eds. Leong and Berka, Marcel Dekker Inc., NY (1992) pp 129 - 148).

### PROTEIN PRODUCTION METHODS

Methods of using the above-described cells are also provided. The proteins produced by the cells may be employed in a variety of methods.

In certain embodiments, the subject methods include: culturing the cell to produce a recombinant protein. The protein will be secreted into the culture medium. As such, certain embodiments of the method include the step of recovering the protein from the culture medium.

A subject fungal cell may be cultured under batch or continuous fermentation conditions. A classical batch fermentation is a closed system, wherein the composition of the medium is set at the beginning of the fermentation and is not subject to artificial alterations during the fermentation. Thus, at the beginning of the fermentation the medium is inoculated with the desired organism(s). In this method, fermentation is permitted to occur without the addition of any components to the system. Typically, a batch fermentation qualifies as a "batch" with respect to the addition of the carbon source and attempts are often made at controlling factors such as pH and oxygen concentration. The metabolite and biomass compositions of the batch system change constantly up to the time the fermentation is stopped. Within batch cultures, cells progress through a static lag phase to a high growth log phase and finally to a stationary phase where growth rate is diminished or halted. If untreated, cells in the stationary phase eventually die. In general, cells in log phase are responsible for the bulk of production of end product.

A variation on the standard batch system is the "fed-batch fermentation" system, which also finds use with the present invention. In this variation of a typical batch system, the substrate is added in increments as the fermentation progresses. Fed-batch systems are useful when catabolite repression is apt to inhibit the metabolism of the cells and where it is desirable to have limited amounts of substrate in the medium. Measurement of the actual substrate concentration in fed-batch systems is difficult and is therefore estimated on the basis of the changes of measurable factors such as pH, dissolved oxygen and the partial pressure of waste gases such as CO₂. Batch and fed-batch fermentations are common and known in the art.

Continuous fermentation is an open system where a defined fermentation medium is added continuously to a bioreactor and an equal amount of conditioned medium is removed simultaneously for processing. Continuous fermentation generally maintains the cultures at a constant high density where cells are primarily in log phase growth.

Continuous fermentation allows for the modulation of one factor or any number of factors that affect cell growth and/or end product concentration. For example, in one embodiment, a limiting nutrient such as the carbon source or nitrogen source is maintained at a fixed rate and all other parameters are allowed to moderate. In other systems, a number of factors affecting growth can be altered continuously while the cell concentration, measured by media turbidity, is kept constant. Continuous systems strive to maintain steady state growth conditions. Thus, cell loss due to medium being drawn off must be balanced against the cell growth rate in the fermentation. Methods of modulating nutrients and growth factors for continuous fermentation processes as well as techniques for maximizing the rate of product formation are known. Methods for recovering protein from growth media by any convenient methods.

A cell engineered in accordance with the above may produce a glycosylated protein that contains fewer or no terminal galactofuranose residues, allowing a co-expressed mannosidase to "trim back" mannose resides on the protein to deglycosylate the protein. If other transferases are expressed in the cell, particularly transferases of mammalian, e.g., human, origin, other residues may be added to the protein, giving the protein a more "human" glycosylation pattern. Such a protein may be less immunoreactive when administered to a human and,
more active, than the same protein produced in an equivalent cell not engineered according to the above.

The ability to modulate glycosylation patterns in proteins produced by filamentous fungi is important for the production of industrial enzymes and therapeutic proteins.

### POLYNUCLEOTIDES, POLYPEPTIDES AND VECTORS

Also provided are polynucleotides, polypeptides and vectors.

A subject polynucleotide may comprise a sequence set forth in any of the figures, a fragment of one of a sequence set forth in any of the figures (where a "fragment" of a polynucleotide is a contiguous sequence of residues at least about 50 nt or 100 nt to at least about 200 nt to 1000 nt, or greater), or a sequence that hybridizes to one of the sequence set forth in any of the figures under stringent hybridization conditions.

A subject polynucleotide may be at least about 70%, at least about 80%, at least about 90%, at least about 95%, or more (i.e. 100%) identical to one of the sequence in the figures. The subject polynucleotide may be a full-length cDNA, for example.

A subject polynucleotide may comprise a sequence set forth in any of the figures, a fragment of one of a sequence set forth in any of the figures (where a "fragment" of a polynucleotide is a contiguous sequence of residues at least about 10 aa or 20 aa to at least about 50 aa to 200 aa, or greater) or a variant polypeptide that has at least about 80%, at least about 90%, or at least about 98% sequence identity to a sequence set forth in the figures.

A subject polypeptide may be naturally-occurring and isolated, recombinant, or not naturally-occurring. The polypeptide may have UDP-galactopyranose mutase activity.

Polynucleotides encoding a subject polypeptide are also provided.

A subject polynucleotide may be present in a vector, for example, a phage, plasmid, viral, or retroviral vector. The vector may be an expression vector for expressing a subject polypeptide in a filamentous fungal cell.

A cell comprising an instant vector is also provided.

### KITS

Also provided are kits. The subject kits at least include one or more of: a subject fungal cell. Other optional components of the kit include: a vector adapted for use in the cell, where the vector contains an expression cassette containing restriction sites (i.e., may contain a multiple cloning site) for inserting a protein coding sequence therein. The various components of the kit may be present in separate containers or certain compatible components may be precombined into a single container, as desired.

In addition to above-mentioned components, the subject kits typically further include instructions for using the components of the kit to practice the subject methods. The instructions for practicing the subject methods are generally recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or subpackaging) etc. The instructions may be present as an electronic storage data file present on a suitable computer readable storage medium, e.g. CD-ROM, diskette, etc. or the actual instructions may not be present in the kit, but means for obtaining the instructions from a remote source, e.g. via the internet, are provided. An example is a kit that includes a web address where the instructions can be viewed and/or from which the instructions can be downloaded. As with the instructions, this means for obtaining the instructions is recorded on a suitable substrate.

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### EXAMPLE 1

Glycan analysis of *Aspergillus niger* strain dgr246ΔGAP *Aspergillus niger* strain dgr246ΔGAP is a derivative of strain dgr246 P2 (Ward, Wilson nd Kodama, 1993, Appl. Microbiol. Biotechnol. 39:738-743) in which the glucoamylase gene *glaA*) has been deleted by replacement with the *A. niger pyrG* gene. Consequently, the *pepA* encoding an aspartic proteinase and *glaA* genes have been deleted from the strain, and the strain as been subjected to several rounds of mutagenesis and screening for improved secretion of oreign proteins.

Strain dgr246ΔGAP was grown for 2 days in CM medium pH 6 (2% sucrose, 0.5% yeast extract). Glycans of secreted proteins were analyzed by DSA-FACE using the methods of Callewaert et al., 2001 (Glycobiology 11:275-281) (Fig. 1). The glycans were observed to be heterogeneous in size, the peak representing the lower molecular weight glycan was of a size consistent with Hex₆GlcNAc₂ whereas the main peak was of a size consistent with Hex₉GlcNAc₂. *In vitro* treatment with α-1,2-mannosidase (panel 3) revealed that only a small fraction of the glycans could be converted to Man₅GlcNAc₂ suggesting that not all terminal residues are α-linked mannoses. Digestion with Jack Bean α-mannosidase (panel 4) confirmed this finding. The non-mannose residue is presumed to be galactofuranose (Gal*f*) as presented in structures 2, 4 & 5 of Fig.1. The exact position of the Gal*f* is unknown. Panel 1 of Fig. 1 shows a series of oligomaltose standards with each peak differing by a single glucose unit. The largest peak in panel 4 runs only 2 glucose units larger than the smallest peak (whereas it would be expected to be 3 glucose units larger). However, the difference between the two major peaks in panel 3 is also less then one glucose unit and it is suggested that the mobility shift caused by Galf addition is not the same as that for mannose addition. In order to demonstrate that the Jack bean mannosidase digested glycan structures were indeed ManGlcNAc₂ and Hex₄GlcNAc₂ (i.e. Gal*f*Man₃GlcNAc₂, see Fig. 1 panel 4 and structures 1 and 2) a partial Jack bean mannosidase digest was performed. This digest revealed that there were indeed 2 peaks between the ManGlcNAc₂ and the Gal*f*Man₃GlcNAc₂ peaks (results not shown).

### EXAMPLE 2

### Overexpression of T. reesei α-1,2-mannosidaseHDEL

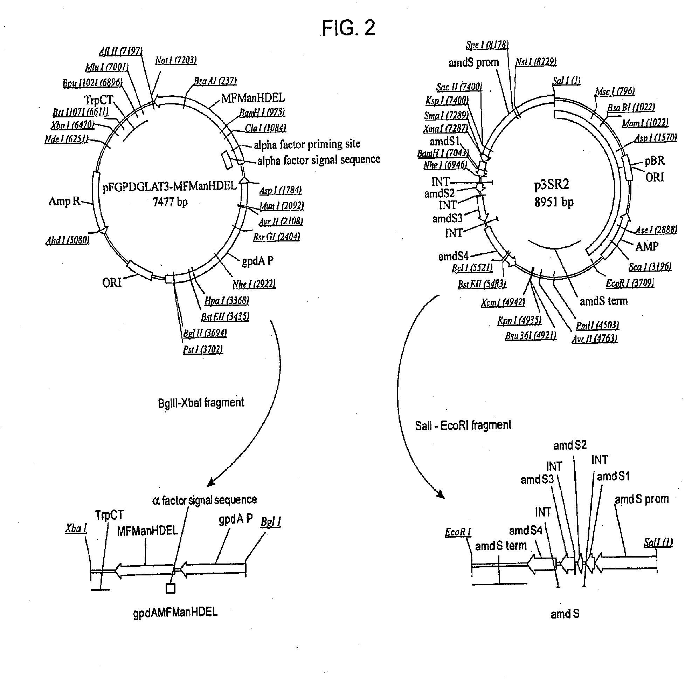

The plasmid pFGPDGLAT3-MFManHDEL was described in Vervecken, 2004 (PhD thesis, University of Ghent). It contains an open reading frame encoding the *Trichoderma reesei* α-1,2-mannosidase with the addition of a C-terminal HDEL amino acid sequence for retention in the endoplasmic reticulum and with the N-terminal signal sequence replaced with the *Saccharomyces* cerevisiae α-mating factor prepro signal sequence as described by Callewaert et al., 2001, FEBS Lett 503:173-178. The mannosidase open reading frame is under control of the *Aspergillus nidulans gpdA* promoter and the *Aspergillus nidulans trpC* terminator (Fig. 2). Plasmid pFGPDGLAT3-MFManHDEL was inserted into *A. niger* using by co-transformation using the *A*. *nidulans amdS* gene as a selection marker (Tilburn et al., 1983, Gene 26:205-221). The plasmid pFGPDGLAT3-MFManHDEL was first cut with *Xba*I/*Bgl*II and the fragment containing the expression cassette was isolated. Plasmid p3SR2 (Tilburn et al., 1983, Gene 26:205-221) was digested with *Sal*I and *Eco*RI and the fragment containing the *amdS* gene was isolated. These fragments were mixed and co-transformed into *A. niger* strain dgr246ΔGAP.

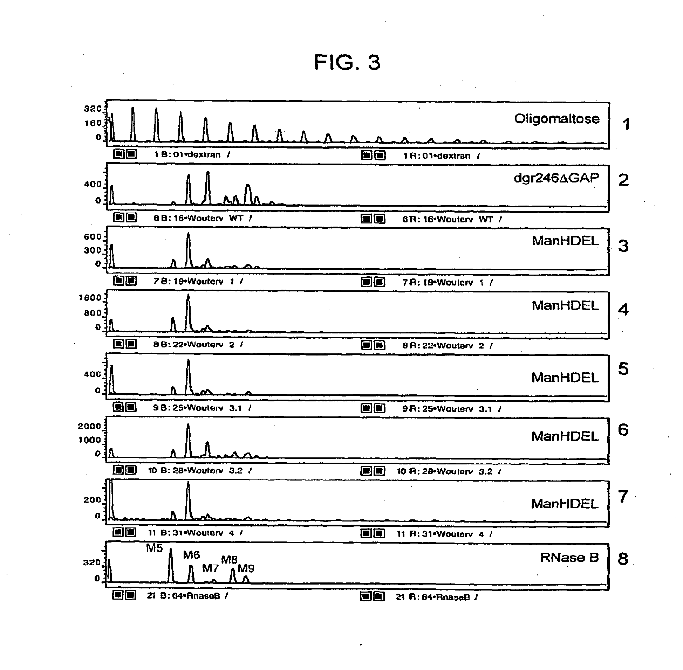

The transformants were selected on minimal medium containing 5 mM acetamide as sole nitrogen source and 10 mM CsCl to reduce background growth. 21 transformants able to grow on acetamide were identified. Transformants were grown in liquid CM medium for 2 days in 100 mL shake flasks. Total secreted proteins were collected and N-glycans were analyzed by DSA-FACE. 13 out of 21 transformants (Fig. 3, panels 3-7 shows examples) showed changes in the glycan pattern compared to strain dgr246ΔGAP (Fig 3, panel 2). A clear shift of high molecular weight to lower molecular weight glycans was observed. The amount of Man₅GlcNAc₂ was increased whereas the most abundant peak was Hex₆GlcNAc₂, probably Gal*f*Man₅GlcNAc₂. Little variation was seen between transformants in the amount of conversion to lower molecular weight glycans. Among the different transformants, transformant 2 (Fig.3, panel 4) had relatively the most Man₅GlcNAc₂ and the least higher structures (Hex₇₋₁₀GlcNAc₂). This transformant dgr246ΔGAP ManHDEL#2 was chosen for further glycan analyses.

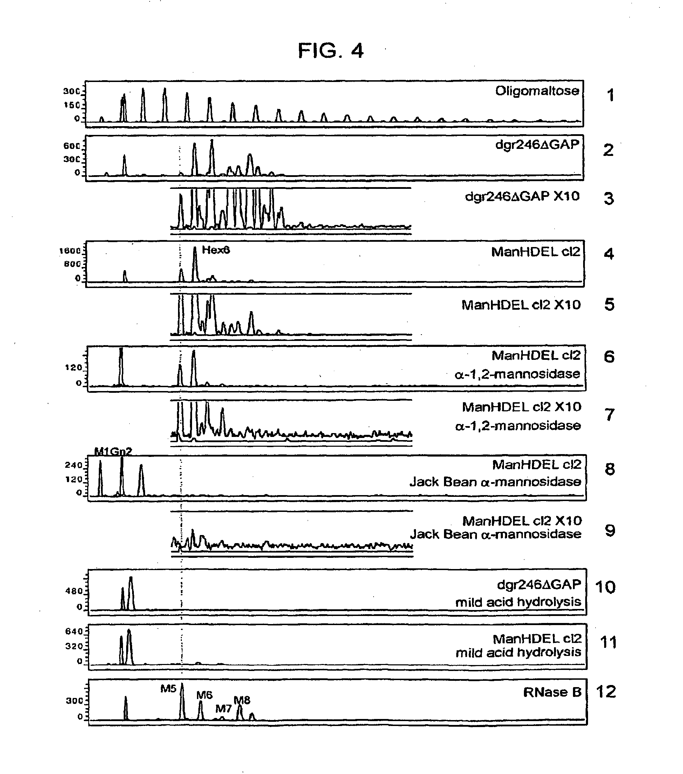

Glycans from dgr246ΔGAP ManHDEL#2 were *in vitro* treated with α-1,2-mannosidase to evaluate the efficiency of the expressed α-1,2-mannosidaseHDEL (Fig. 4). The Hex₆GlcNAc₂ peak of dgr246ΔGAP ManHDEL#2 (called ManHDEL cI2 in Fig 4) was not sensitive to *in vitro* mannosidase treatment. Minor differences in the glycan profile between the *in vitro* digested and the native glycans from dgr246ΔGAP ManHDEL#2 could only be observed if the electropherogram was magnified 10 times (Fig. 4, panel 4 & 5 versus 6 & 7). From these results it was concluded that the *in vivo* expression of the α-1,2-mannosidaseHDEL resulted in complete trimming of the glycans. Treatment with Jack bean α-mannosidase revealed that, as in the glycans from strain dgr246ΔGAP, a major fraction of the glycans contain a non α-Man residue, most probably Gal*f* (Fig.4, panel 8 & 9).

### EXAMPLE 3

### UDP-Galp mutase gene

UDP-Gal*p* mutase genes have been cloned from *Aspergillus fumigatus* (Bakker et al., 2005, Biol Chem 386:657-661) and *Aspergillus nidulans* (Hans Bakker, unpublished data). A draft sequence of the *A. nidulans* UDP-Gal*p* mutase gene was obtained from Dr. Hans Bakker (Medizinische Hochschule Hannover). The *A. nidulans* sequence (Fig. 12) was used to search for homologs in an *A. niger* genomic sequence database supplied by Integrated Genomics, Inc. (Chicago, IL).

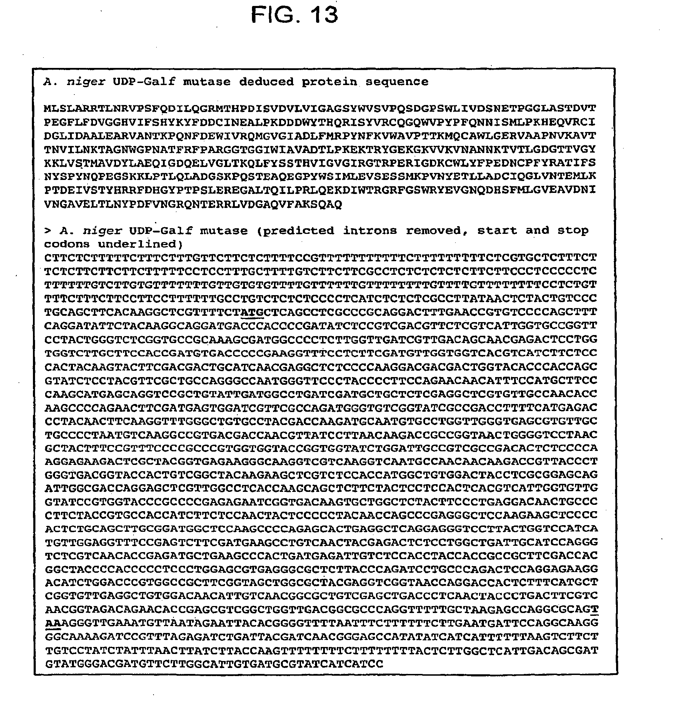

The unconfirmed DNA sequence and predicted amino acid sequence of one *A. niger* UDP-Gal*p* mutase homolog was obtained from the *A. niger* genome database are shown in Figs. 13-15. The open reading frame of the gene appears to be full-length, although errors are possible.

A pair of primers (CCGAAGCTTATGCTCAGCCTCGCCCG; SEQ ID NO:12 and CGCGGATCCTTACTGCGCCTGGCTCTTAG; SEQ ID NO:13) were designed to amplify the open reading frame from start to stop codon by PCR. A PCR reaction using genomic DNA from strain dgr246ΔGAP as template was performed using these primers. A fragment of approximately the expected length was obtained and was purified by agarose gel electrophoresis, cloned into the pCR2.1-TOPO cloning vector (Invitrogen, Carlsberg, CA) according to the manufacturer's instructions, and sequenced. Some sequence differences were identified. Introns and exons were identified using the Genewise program and by comparison with a publicly available *A. niger* ESTs and the deduced *A. nidulans* protein sequence. The corrected sequences are set forth in Fig. 18.

### EXAMPLE 4

### Construction of a deletion vector for the A. niger UDP-Galp mutase

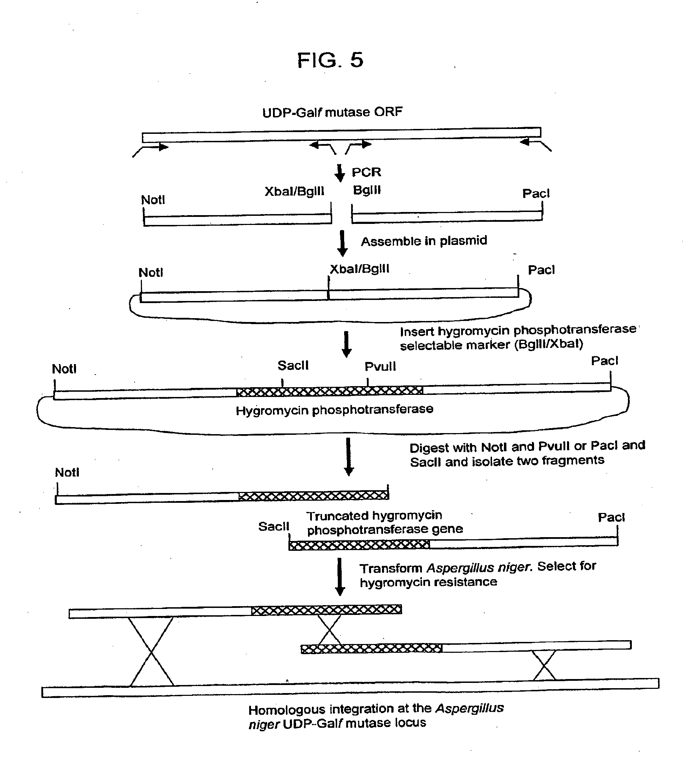

To construct a deletion vector for the *A. niger* UDP-Gal*p* mutase a strategy (illustrated in Fig. 5) was designed in which 100 bp in exon 5, close to the center of the open reading frame would be replaced with a hygromcin-resistance gene. To accomplish this, a fragment of the first 944 bp of the gene (numbering begins at the start codon) was amplified by PCR using the following primers and inserted into the pCR2.1-TOPO vector. GCAGCGGCCGCATGCTCAGCCTCGCCCG (SEQ ID NO:14; *Not*I site is underlined) and GGCAGA**TCTAGA**TTAGGGGCAGCAACACGCTC (SEQ ID NO:15; *Bgl*II site is underlined, *Xba*I site is in bold).

Similarly, a fragment containing bp 1044 -1969 was obtained by PCR using the following primers and cloned into pCR2.1-TOPO:
GGCAGATCTGGCGCGCCATCTGGATTGCCGTCGCCG (SEQ ID NO:16; *Bgl*II site is underlined) and GCC**TCTAGA**TTAATTAATTACTGCGCCTGGCTCTTACCAAA (SEQ ID NO:17; *Xba*I site is in bold, *Pac*I site is underlined).

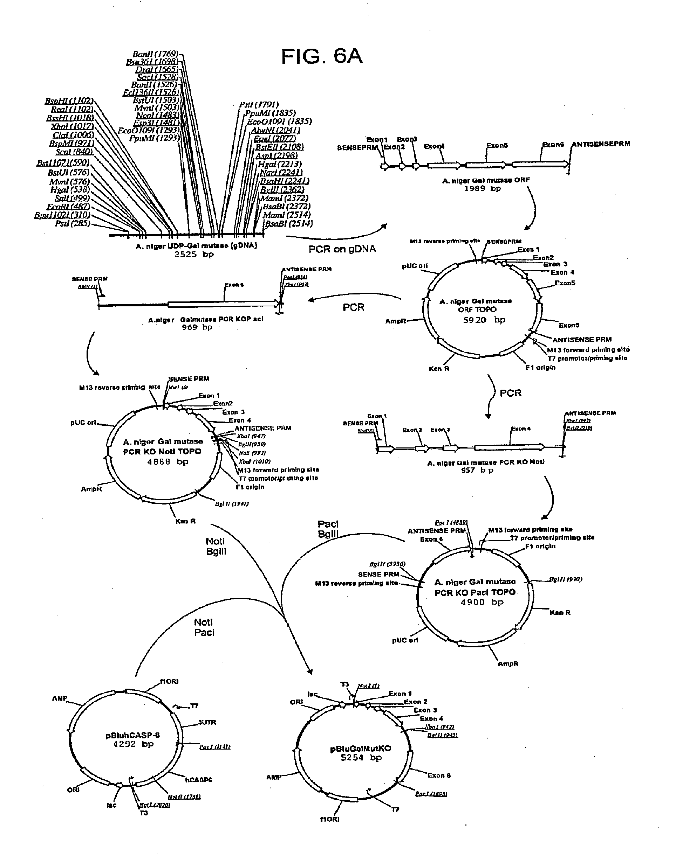

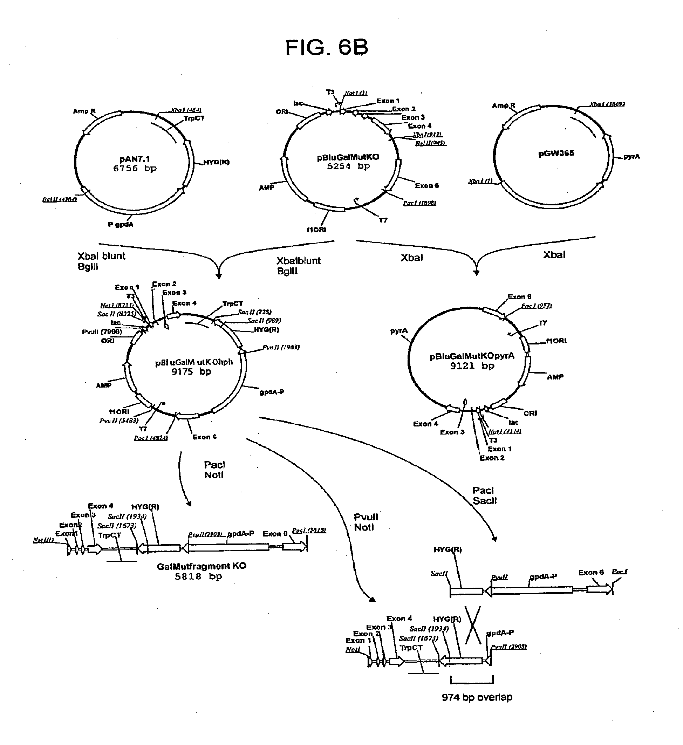

The two fragments were isolated by restriction digest and ligated into a vector with unique *Pac*I, *Not*I and *Bgl*II sites (pBluhCASP-6) to create pBluGalMutKO. See construction scheme Fig. 6. The *Escherichia coli* hygromycin phosphotransferase gene under control of the *Aspergillus nidulans gpdA* promoter and *A. nidulans trpC* terminator was obtained from plasmid pAN7-1 (Punt et al., 1987, Gene 56:117-124) and inserted into pBluGalMutKO creating pBluGalMutKOhph. Digestion with *Pac*I/*Sac*II or *Not*I/*Pvu*II allows the isolation of two fragments that on their own cannot lead to *A. niger* transformants via random integration because each fragment has only a truncated version of the hygromycin phosphotransferase gene. The fragments must recombine with each other to create a functional hygromycin phosphotransferase gene. The overlap of identical sequence between the two fragments is 974 bp.

### EXAMPLE 5

### Transformation of A. niger

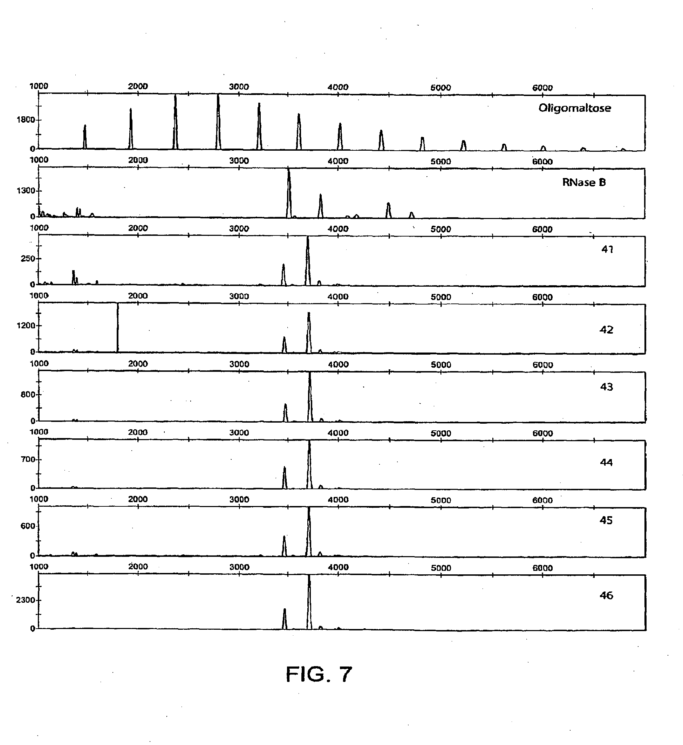

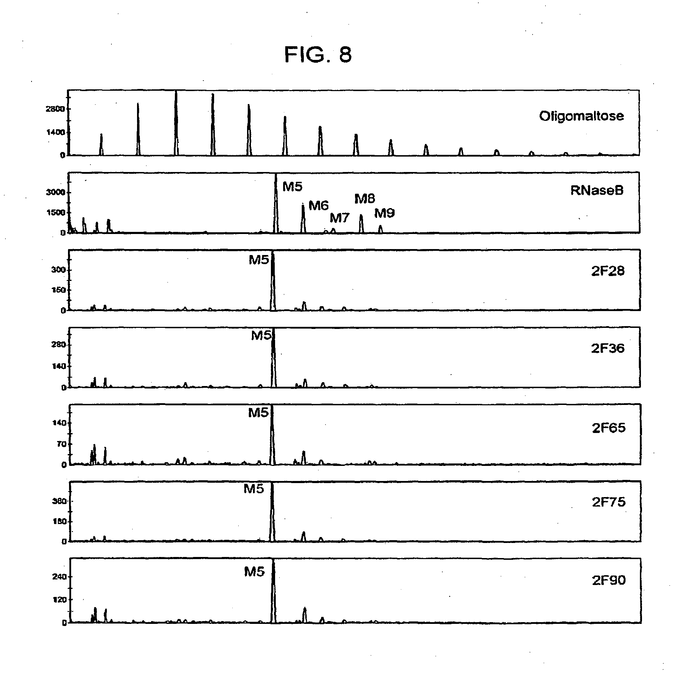

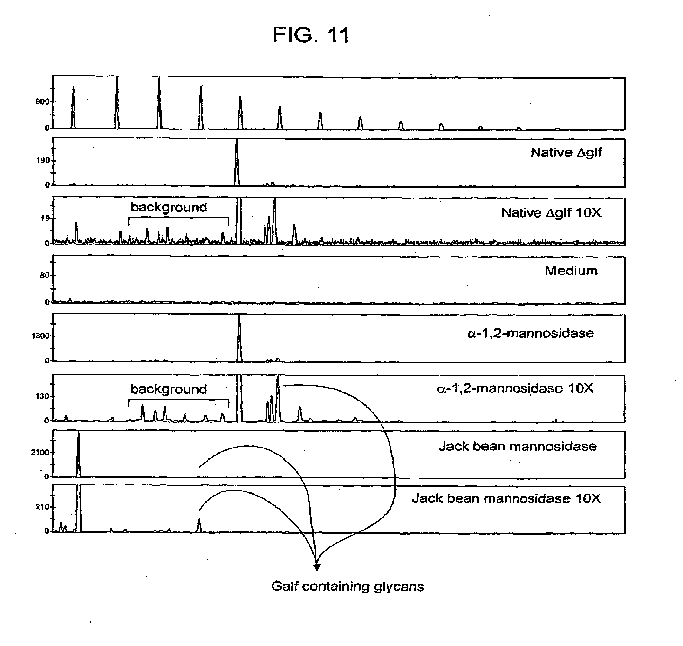

The plasmid pBluGalMutKOhph was cut with *Pac*I and *Sac*II or *Not*I and *Pvu*II. The desired fragments were isolated after agarose gel electrophoresis (see Fig. 6), mixed and transformed to *A. niger* strain dgr246ΔGAP ManHDEL transformant #2. 134 hygromycin B resistant clones were obtained. These were reselected by streaking on hygromycin B plates and single clones were isolated. Transformants that continued to show hygromycin B resistance were grown in shake flasks and the N-linked glycans present on the secreted proteins were analyzed via DSA-FACE. 129 of the clones showed no apparent change in N-glycan pattern (Fig. 7). However, on DSA-FACE analysis five transformants (2F28, 2F36, 2F65, 2F75 and 2F90) that grew slower than the parental strain on plates and produced less spores were identified that showed a dramatic decrease in the Hex₆ peak, so that the most abundant peak was now Hex₅GlcNAc₂ (Fig. 8). This peak was demonstrated to be Man₅GlcNAc₂ by digestion with Jack bean mannosidase that was able to trim the structure back to ManGlcNAc₂ (Fig. 11, lower two panels).

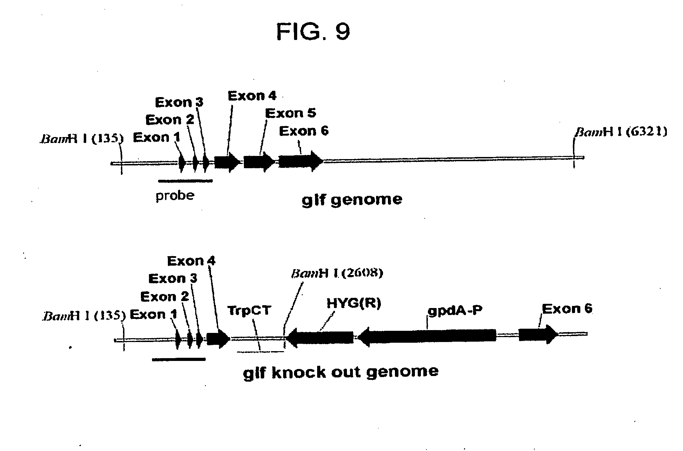

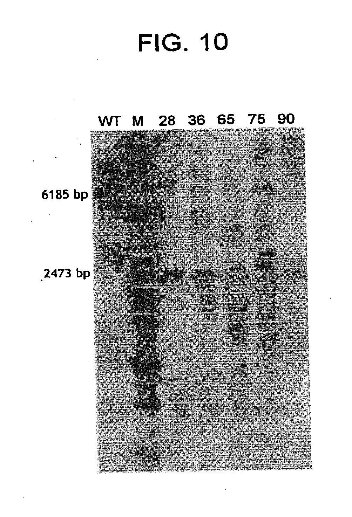

To confirm that the UDP-Gal*p* mutase gene had been deleted in the five transformants that showed altered glycan patterns Southern analysis was performed. Genomic DNA was isolated, digested with *Bam*HI, separated by agarose gel electrophoresis, blotted to a membrane and probed with a radioactively labeled *BspH*I/*Sph*I fragment of the UDP-Gal*p* mutase gene (5' end). This fragment would be expected to hybridize to a fragment of 6185 bp in the parental strain and to a fragment of 2473 bp in a UDP-Gal*p* mutase deletion strain (see Fig. 9). In 4 of the 5 candidate deletion strains only the expected band of 2473 bp was observed and the 6185bp band of the parental strain was absent (Fig. 10).

From the N-glycan profiles (Fig. 8) it can be deduced that, although the dominant glycan form by far is Man₅GlcNAc₂, not all glycans are trimmed to Man₅GlcNAc₂. therefore mannosidase treatment of the N-glycans of clone 2F65 was performed (see Fig. 11) to see whether these other glycans do still contain Gal*f*. It should be noted that the glycans presented in this Figure are from a different culture than used for Figure 8. Consequently, the relative amount of non-Man₅GlcNAc₂ structures is lower. The conclusions that can be drawn from Fig. 11 are that the non-Man₅GlcNAc₂ glycans cannot be hydrolyzed with α-1,2-mannosidase. After treatment with Jack bean mannosidase two peaks appear: ManGlcNAc₂ and a structure that runs at the same position as the remnant peak from the dgr246ΔGAP glycans treated with Jack bean mannosidase. These conclusions suggest the possibility that there may be some residual terminal Gal*f* present on the glycans.

### EXAMPLE 6

### A second UDP Galp mutase gene of A. niger

A second homolog of the UDP-Gal*p* mutase gene was identified by searching the *Aspergillus niger* genome database. It is possible that this gene is responsible for an activity that leads to residual Gal*f* residues on glycans after deletion of the first UDP-Galp mutase gene. Only a portion of the open reading frame is present within this sequence. This gene may be inactivated using methods similar to those discussed above.

### EXAMPLE 7

### Antibody production in Aspergillus niger strain 2F36

An expression vector is constructed and contains the following components. The promoter and terminator regions from the *Aspergillus niger* glucoamylase (*glaA*) gene are operably linked to an open reading frame encoding a fusion protein comprising, from the amino terminus, the *A. niger* glucoamylase signal sequence, prosequence, catalytic core region and linker region followed by a human IgG1 antibody heavy chain. A KexB cleavage site (Lys Arg) is located after the glucoamylase linker and before the antibody heavy chain so that cleavage after the Arg residue releases the antibody heavy chain from the fusion protein. A second expression vector is constructed and contains the following components. The promoter and terminator regions from the *Aspergillus niger* glucoamylase (*glaA*) gene are operably linked to an open reading frame encoding a fusion protein comprising, from the amino terminus, the *A. niger* glucoamylase signal sequence, prosequence, catalytic core region and linker region followed by a human antibody light chain. A KexB cleavage site (Lys Arg) is located after the glucoamylase linker and before the antibody light chain so that cleavage after the Arg residue releases the antibody light chain from the fusion protein.

The two expression vectors are introduced into *A. niger* cells by co-transformation. A selectable marker for transformation is either present on at least one of the two expression vectors or may be encoded by a third DNA molecule included during the co-transformation process.

Transformants are obtained and are grown in suitable liquid culture medium. Supernatant samples containing secreted proteins are recovered from the cultures and human antibody is purified. DSA-FACE analysis is performed to determine the size and structure of the N-linked glycan attached to the antibody. It is expected that the glycan is predominantly of the Man₅GlcNAc₂ form. Further details of the construction and use of the vectors described in this example may be found in WO03089614.

## Claims

1. A recombinant filamentous fungal cell comprising a nuclear genome comprising an inactivated UDP-galactopyranose mutase (UDP-gal*p* mutase) gene, wherein said fungal cell further comprises a recombinant nucleic acid for expression of a secreted protein in said cell.

2. The fungal cell of claim 1, wherein said UDP-galP mutase gene contains a deletion, an insertion, or a rearrangement.

3. The fungal cell of claim 1, wherein said fungal cell is an *Aspergillus* cell.

4. The fungal cell of claim 1, wherein said fungal cell is an *Aspergillus niger* cell or an *Aspergillus oryzae* cell.

5. The fungal cell of claim 1, wherein said secreted protein is a glycosylated protein

6. The fungal cell of claim 1, wherein said secreted protein is heterologous to said cell.

7. The fungal cell of claim 1, wherein said secreted protein is native to said cell.

8. The fungal cell of claim 1, wherein said secreted protein is a therapeutic protein, an antibody protein or an enzyme.

9. The fungal cell of claim 8, wherein said enzyme is an amylase, protease, cellulase, xylanase or phytase.

10. The fungal cell of claim 1, wherein said nuclear genome comprises at least two inactivated UDP-galactopyranose mutase genes.

11. The fungal cell of claim 1, wherein said fungal cell further comprises a recombinant nucleic acid for expression of a mannosidase.

12. A method of producing the secreted protein encoded by the recombinant nucleic acid, comprising culturing the cell of any one of claims 1 to 11 in culture medium under conditions suitable to produce said secreted protein.

13. The method of claim 12, further comprising recovering said secreted protein from said culture medium.

14. A method of making the cell of claim 1, comprising a) introducing a recombinant nucleic acid into a filamentous fungal cell so that said recombinant nucleic acid recombines with UDP-galactopyranose mutase gene of said genome of said cell thereby inactivating said gene; and
b) introducing a second recombinant nucleic acid into said cell that provides for expression of said secreted protein.

15. The method of claim 14, wherein said nucleic acid inserts into said UDP-galactopyranose mutase gene.

16. The method of claim 14, wherein said nucleic acid deletes at least a portion of said UDP-galactopyranose mutase gene.

## Patentansprüche

1. Rekombinante filamentöse Pilzzelle, umfassend ein Kerngenom, umfassend ein inaktiviertes UDP-Galactopyranose-Mutase-(UDP-gal*p*-Mutase)-Gen, wobei die Pilzzelle weiterhin eine rekombinante Nucleinsäure für Expression eines sekretierten Proteins in der Zelle umfasst.

2. Pilzzelle nach Anspruch 1, wobei das UDP-galP-Mutase-Gen eine Deletion, eine Insertion oder eine Umlagerung enthält.

3. Pilzzelle nach Anspruch 1, wobei die Pilzzelle eine *Aspergillus*-Zelle ist.

4. Pilzzelle nach Anspruch 1, wobei die Pilzzelle eine *Aspergillus*-*niger-*Zelle oder eine *Aspergillus*-*oryzae-*Zelle ist.

5. Pilzzelle nach Anspruch 1, wobei das sekretierte Protein ein glycosyliertes Protein ist.

6. Pilzzelle nach Anspruch 1, wobei das sekretierte Protein heterolog für die Zelle ist.

7. Pilzzelle nach Anspruch 1, wobei das sekretierte Protein nativ für die Zelle ist.

8. Pilzzelle nach Anspruch 1, wobei das sekretierte Protein ein therapeutisches Protein, ein Antikörper-Protein oder ein Enzym ist.

9. Pilzzelle nach Anspruch 8, wobei das Enzym eine Amylase, Protease, Cellulase, Xylanase oder Phytase ist.

10. Pilzzelle nach Anspruch 1, wobei das Kerngenom mindestens zwei inaktivierte UDP-Galactopyranose-Mutase-Gene umfasst.

11. Pilzzelle nach Anspruch 1, wobei die Pilzzelle weiterhin eine rekombinante Nucleinsäure für Expression einer Mannosidase umfasst.

12. Verfahren zum Erzeugen des sekretierten Proteins, codiert durch die rekombinante Nucleinsäure, umfassend Kultivieren der Zelle nach einem der Ansprüche 1 bis 11 in Kulturmedium unter Bedingungen, geeignet, um das sekretierte Protein zu erzeugen.

13. Verfahren nach Anspruch 12, weiterhin umfassend Gewinnen des sekretierten Proteins aus dem Kulturmedium.

14. Verfahren zum Herstellen der Zelle nach Anspruch 1, umfassend a) Einführen einer rekombinanten Nucleinsäure in eine filamentöse Pilzzelle, so dass die rekombinante Nucleinsäure mit einem UDP-Galactopyranose-Mutase-Gen des Genoms der Zelle rekombiniert, dadurch Inaktivieren des Gens; und
b) Einführen einer zweiten rekombinanten Nucleinsäure in die Zelle, die für Expression des sekretierten Proteins sorgt.

15. Verfahren nach Anspruch 14, wobei die Nucleinsäure in das UDP-Galactopyranose-Mutase-Gen insertiert.

16. Verfahren nach Anspruch 14, wobei die Nucleinsäure zumindest einen Teil des UDP-Galactopyranose-Mutase-Gens deletiert.

## Revendications

1. Cellule de champignon filamenteux recombinante comprenant un génome nucléaire comprenant un gène d'UDP-galactopyranose mutase (UDP-gal*p* mutase) inactivé, où ladite cellule fongique comprend en outre un acide nucléique recombinant pour une expression d'une protéine sécrétée dans ladite cellule.

2. Cellule fongique selon la revendication 1, dans laquelle ledit gène d'UDP-galP mutase contient une délétion, une insertion ou un réarrangement.

3. Cellule fongique selon la revendication 1, où ladite cellule fongique est une cellule d'*Aspergillus.*

4. Cellule fongique selon la revendication 1, où ladite cellule fongique est une cellule d'*Aspergillus niger* ou une cellule d'*Aspergillus oryzae*.

5. Cellule fongique selon la revendication 1, dans laquelle ladite protéine sécrétée est une protéine glycosylée.

6. Cellule fongique selon la revendication 1, dans laquelle ladite protéine sécrétée est hétérologue pour ladite cellule.

7. Cellule fongique selon la revendication 1, dans laquelle ladite protéine sécrétée est naturelle pour ladite cellule.

8. Cellule fongique selon la revendication 1, dans laquelle ladite protéine sécrétée est une protéine thérapeutique, une protéine d'anticorps ou une enzyme.

9. Cellule fongique selon la revendication 8, dans laquelle ladite enzyme est une amylase, une protéase, une cellulase, une xylanase ou une phytase.

10. Cellule fongique selon la revendication 1, dans laquelle ledit génome nucléaire comprend au moins deux gènes d'UDP-galactopyranose mutase inactivés.

11. Cellule fongique selon la revendication 1, où ladite cellule fongique comprend en outre un acide nucléique recombinant pour une expression d'une mannosidase.

12. Procédé pour la production de la protéine sécrétée codée par l'acide nucléique recombinant, comprenant la culture de la cellule selon l'une quelconque des revendications 1 à 11 dans un milieu de culture dans des conditions appropriées pour produire ladite protéine sécrétée.

13. Procédé selon la revendication 12, comprenant en outre la récupération de ladite protéine sécrétée à partir dudit milieu de culture.

14. Procédé pour la fabrication de la cellule selon la revendication 1, comprenant a) l'introduction d'un acide nucléique recombinant dans une cellule de champignon filamenteux de sorte que ledit acide nucléique recombinant se recombine avec un gène d'UDP-galactopyranose mutase dudit génome de ladite cellule inactivant ainsi ledit gène; et
b) l'introduction d'un deuxième acide nucléique recombinant dans ladite cellule qui permet l'expression de ladite protéine sécrétée.

15. Procédé selon la revendication 14, dans lequel ledit acide nucléique s'insert dans ledit gène d'UDP-galactopyranose mutase.

16. Procédé selon la revendication 14, dans lequel ledit acide nucléique délète au moins une portion dudit gène d'UDP-galactopyranose mutase.
